**Europäisches Patentamt**

**European Patent Office**

**Office européen des brevets**

(19)

(11) Veröffentlichungsnummer: **0 093 272 B1**

(12)

# EUROPÄISCHE PATENTSCHRIFT

(45) Veröffentlichungstag der Patentschrift:
26.02.86

(51) Int. Cl.⁴: **A 61 B 5/14, A 61 M 1/36**

(21) Anmeldenummer: 83103397.2

(22) Anmeldetag: 07.04.83

(54) **Entlüftungsventil als Blutsperre.**

(30) Priorität: 03.05.82 DE 3216472

(43) Veröffentlichungstag der Anmeldung:
09.11.83 Patentblatt 83/45

(45) Bekanntmachung des Hinweises auf die Patenterteilung:
26.02.86 Patentblatt 86/9

(84) Benannte Vertragsstaaten:
AT BE CH DE FR GB IT LI LU NL SE

(56) Entgegenhaltungen:
FR - A - 2 084 836
FR - A - 2 123 689
GB - A - 1 343 394
US - A - 3 906 958
US - A - 4 266 558

Prospekt Nr. 6, 135.05.75/1, Seite 31
"Braun-Einmal-Produkte für die praxisgerechte
Therapie", B. Braun Melsungen AG, 3508 Melsungen

(73) Patentinhaber: INTERMEDICAT GMBH,
Gerliswilstrasse 74, CH-6020 Emmenbrücke (CH)

(72) Erfinder: Sinning, Karl-Heinz, Fichtenweg 10,
D-3503 Lohfelden 1 (DE)

(74) Vertreter: von Kreisler, Alek, Dipl.-Chem. et al,
Deichmannhaus am Hauptbahnhof, D-5000 Köln 1 (DE)

LIBER, STOCKHOLM 1986

## Beschreibung

Die Erfindung betrifft ein Entlüftungsventil als Blutsperre für medizinische Leitungen oder Kanülen, mit mindestens einem kapillaren Entlüftungskanal.

Derartige Entlüftungsventile dienen dazu, medizinische Geräte bei Blutfüllung zu entlüften, wobei das Blut nach Eintritt in das Entlüftungsventil koaguliert und somit den weiteren Blutfluß anhält. Die bekannten Entlüftungsventile werden als Blutfängerstopfen bezeichnet und bestehe, jeweils aus einem Halter, in dem sich ein Feinfilter befindet. Als Filter werden offenporige geschäumte bzw. gesinterte einteilige Stopfen verwandt. Wenn Blut durch die Leitung bzw. Kanüle durch den Filter strömt, entweicht Luft durch das Filter hindurch. Nachdem das Blut den Filter erreicht hat, koaguliert es in dessen engen Kanälen. Ein solches Entlüftungsventil ist Z.B. aus dem Prospekt "Braun-Einmal-Produkte für praxisgerechte Therapie", Nr G 135. 05. 75/1, seite D-3508 Melsunger, ersichrlich.

Nachteilig ist bei den bekannten Filtern, daß sie aus mehreren Einzelteilen zusammenges somit aufwendig in der Herstellung sind. Noch schwerwiegender ist allerdings der Nachteil, daß ein blutbenetzter Filter nicht kontaminationssicher ist. Durch Berührungen mit der Hand oder durch die Umgebungsluft können Bakterien in den Filter und durch diesen in das Blut gelangen.

Der Erfindung liegt die Aufgabe zugrunde, ein Entlüftungsventil der eingangs genannten Art zu schaffen, das mit einfache, Mitteln zu realisieren ist, eine schnelle Entlüftung bei der Blutfüllung des medizinischen Gerätes gewährleistet und kontaminationssicher ist.

Zur Lösung dieser Aufgabe ist erfindungsgemäß vorgesehen, daß ein rohrförmiges Teil, dessen Inneres mit der Leitung bzw. Kanüle verbuden ist, durch eine Kappe verschlossen ist, da ß eine von zwei sich koaxial mit dem rohrförmigen Teil bzw. mit der Kappe erstreckenden, zusammenwirkenden umlaufenden Flächen des Rohrstüs und der Kappe mindestens eine schraubenförmioge Nut aufweist, die eine Verbindung zwischen dem Inneren des rohrförmigen Teiles und der Außenluft herstellt, und daß mindestens eine der beiden Flächen ein das Blut koagulierendes Material aufweist.

Durch die schraubenförmige bzw. wendelförmige Nut wird die Entlüftung des Gerätes ermöglicht. In diese Nut tritt nach einer Punktion Luft ein. Durch den Venendruck wird die Luft verdrängt und das nachströmencd Blut mindert durch seine Adhäsionswirkung und Kohäsionswirkung den Venendruck derart, daß nach Füllen einiger Windungen der Nut der Venendruck abgebaut ist. Das schnelle Koagulieren des Blutes wird durch das koagulierende Material erreicht, das an den den wendenförmigen Kapillarkanal begrenzenden Wänden angeordnet ist. Das koagulierte Blut und die weiteren blutfreien Windungen der Nut bewirken den gewüschten Kontaminationsschutz. Bei dem Einströmen des Blutes in die enge wendelförmige Nut wird der Blutstrom abgebremst.

Um den Gerinnungsprozeß des Blutes rasch in Gang zu setzen, befindet sich an der Oberfläche mindestens einer der zur Bildung les Kapillarkanales zusammenwirkende Flächen ein Werkstoff, der das Gerinnungsgeschehen über die sogenannte Kontaktaktivierung induziert. Es sind eine Reihe von Mineralien bekannt, die eine Gerinnung bei Blutkontakt auslösen, z.B. Kaolin, Celit und Bentonit. Auch langkettige Fettsäuren sind geeignet Substanzen,die gerinnugsinduzierendes Verhalten zeigen. Ebenso können Proteine wie Kollagen und Elastin zur spontanen Gerinnung benutzt werden. Ferner lassen sich auch verschiedene Kunststoffe verwenden, die eine geringe Blutverträglich keit aufweisen. Auch Glas ist als koagulationsförderndes Wandmaterial geeignet.

Eine relativ einfache Möglichkeit, das Blut zur Gerinnung zu bringen, besteht darin, in die Nut eine Kalziumchlo ridlösung, ein flüssiges Detergez oder einen flüssigen Ionenaustauscher einzuschließen oder die zusammenwirken den Flächen damit zu beschichten.

Die Tiefe der Nut kann in der Größenordnung von 1/10mm liegen oder auch wesentlich darunter. Wichtig ist, daß die wendelförmige Nut ein äußerst geringen Querschnitt hat. Wenn die Nut als mehrgängiges Gewinde ausgebildet ist, werden mehrere parallele Kapillarkanäle gebildet. In diesem Fall kann der Nutquerschnitt zusätzlich noch verkleinert werden.

Vorzugsweise sind die zusammenwirkenden Flächen konisch ausgebildet, um eine feste Klemmung der Kappe an dem rohrförmige Teil zu bewirken. In diesem Fall ergibt sich eine besonders einfache Montage der beiden Teile aneinander. Ferner können beide Teile zum Anschluß eines anderen Gerätes an das rohrförmige Teil schnell und einfach gelöst werden.

Gemäß einer bevorzugten Ausführungsform der Erfindung weist die Kappe einen das rohrförmige Teil mit radialem Abstand umgebenden Mantel auf. Dieser Mantel kann mit Verriegelungselemenren zur Verriegelung an der Außenseite des rohrförmiger Teiles ausgestatter sein. Er bewirkt einen zuzätzlichen Kontaminationsschutz.

Im folgenden werden unter Besugnahme auf die Zeichnungen Ausführungsbeispile der Erfindung näher erläutert. Es zeigen:

Fig. 1 einen Längsschnitt durch eine Kanüle mit auseinandergenommenem Entlüftungsventil,

Fig. 2 einen Längsschnitt durch das Entlüftungsventil der Fig. 1 im montiertem Zustand und

Fig. 3 einen Längsschnitt durch eine andere Ausführungsform eines Entlüftungsventils.

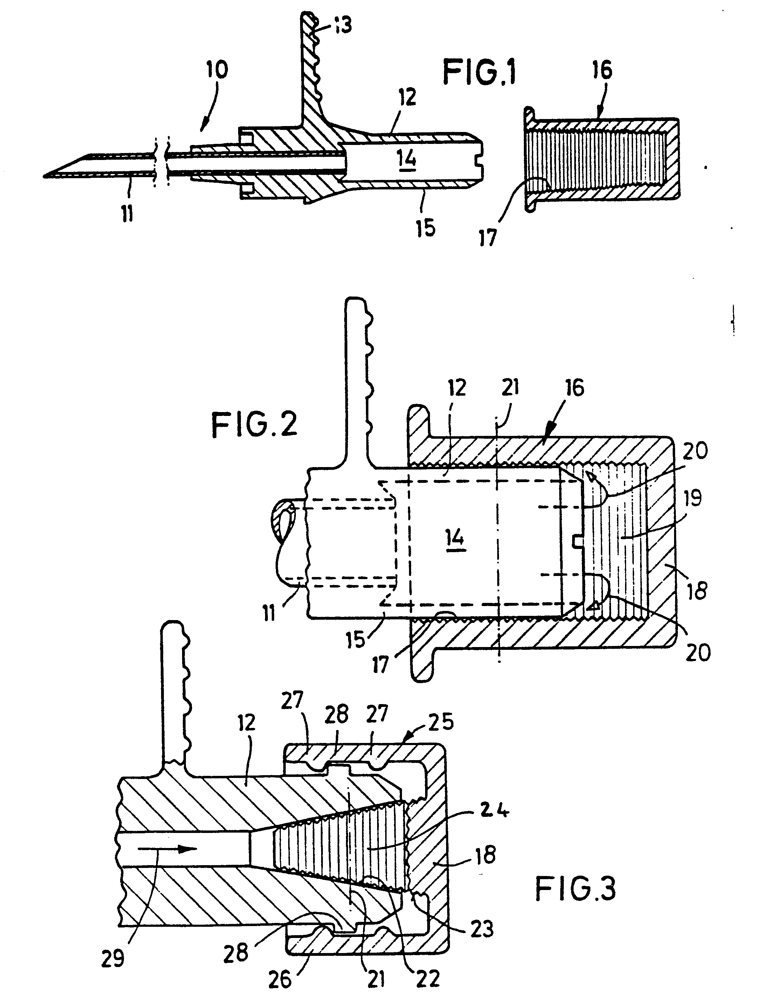

Die Punktionskanüle 10 nach Fig. 1 weist am rückwärtigen Ende eines Metallrohres 11 ein Anschlußstück 12 in Form eines rohrförmigen Teiles auf, dessen Innenraum mit dem Innern des Rohres 11 in Verbindung steht. Dem rohrförmigen Teil 12 ist ein Handgriff 13 zur Handhabung der Punktionskanüle angeformt.

Das rohrförmige Teil 12 weist einen im wesentlichen zylindrischen, nur zum rückwärtigen Ende offenen Innenraum 14 auf.Die Außenfläche 15 des rohrförmigen Teiles ist leicht konisch abgeschrägt.

Auf das rückwärtige Ende des rohrförmigen Teiles 12 wird die Kappe 16 aufgeschoben. Die leicht konische Innenfläche 17 dieser Kappe 16 weist eine kontinuierliche umlaufende Nut nach Art eines Innengewindes auf. Die Tiefe der Nut beträgt eta 1/10 mm und die Ganghöhe ca. 0,2 bis 0,4 mm. Wenn die Kappe 16 gemäß Fig. 2 auf das rohrförmige Teil 15 aufgeklermmt ist, besteht zwischen dem rückwärtigen Ende des rohrförmigen Teiles 12 und der Stirnwand 18 der Kappe ein Hohlraum 19g.

Während das rohrförmige Teil 12 aus undurchsichtigem Kunststoff besteht, besteht die Kappe 16 aus einem durchsichtigen Material. Die mit der wendelförmigen Nut versehene Innenfläche 17 ist mit einem Koagulationsmitte geträkt bzw. besprüht oder sie besteht selbst aus einem koagulierenden Material.

Das venöse Blut strömt durch den Innenraum 14 und von dort in den Hohlraum 19 im Innern der Kappe 16. Von dort gelangt das Blut in Richtung der Pfeile 20 in die kapillare Nut, um in dieser zwischen den Wänden 15 und 17 zurückzufließen. Infolge der Durchsichtigkeit der Kappe l6 Kann die Blutgrenze 21 optisch leicht festgestellt werden. Der Bereich links von der Blutgrenze 21 wirkt weiterhin als Kontaminationsschutz. Verunreinigungen und Bakterien müssen, um an die Blutgrenze 21 zu gelangen, von außen her die wendelförmige Nut Über einen erheblichen Abstand durchlaufen.

Bei dem Ausführungsbeispiel der Fig. 3 ist der Innenraum des rohrförmigen Teiles 12 an seinem Ende konisch erweitert. In diese konische glatte Innenfläche 22 ist der konische Ansatz 24 der Kappe 25 passend eingesetzt. Die. Außenfläche 23 des konischen Ansatzes 24 weist die wendelförmige Nut auf. Der konische Ansatz 24 steht frei von der rückwärtigen Stirnwand 18 ab. Er ist auf einem Teil seiner Länge von einem zylindrischen Mantel 26 umgeben, der das rückwärtige Ende der rohrförmigen Teiles 12 mit radialem Abstand umgibt. Der Mantel 26 weist an seiner Innenseite gewindeartige Stege 27 auf, die mit zwei nach entgegengesetzten Richtungen von dem rohrförmingen Teil 12 nach außen abstehenden Zapfen 28 zusammenwirken, so daß die Kappe 25 sicher auf dem rohrförmigen Teil 12 festgehalten wird, ohne daß der Luftdurchgang durch den Ringraum zwischen dem Mantel 26 und dem rohrförmigen Teil 12 behindert würde.

Nach einer Punktion baut sich der Druck im Innern des rohrförmigen Teiles 12 aus Richtung des Pfeiles 29 auf. Dabei wird Luft durch die wndelförmigen Nut und den. Ringraum zwischen dem Mantel 26 und dem Teil 12 ausgetrieben. Nachdem das Blut einen Teil der wendelförmigen Nut durchlaufen hat, gerinnt es in dieser an der Blutgrenne 21. Der hinter der Blutgrenze 21 liegende Bereich der Nut und der von der Kappe 25 umschlossene Bereich dienen als Kontaminationsschutz. Sowohl das Teil 12 als auch der Mantel 26 der Kappe 25 sind durchsichtig.

Ein besonderer Vorteil des Entlüftungsventils besteht darin, daß allein die Kappe in besondere Weise, nämlich durch Anbringung der wendelförmigen Nut, gestaltet sein muß, während das rohrförmige Teil keine spezielle Gestaltunq erfordert.

**Patentansprüche**

1. Entlüftungsventil als Blutsperre für medizinische Leitungen oder Kanülen/mindesten einem kapillaren Entlüftungskanal, dadurch gekennzeichnet, daß ein rohrförmiges Teil (12), dessen Inneres (14) mit der Leitung bzw. Kanüle verbunden ist, durch eine Kappe (16;25) verschlosse ist, daß eine von zwei sich koaxial mit dem rohrförmigen Teil bzw. mit der Kappe erstrekkenden, zusammenwirkenden umlaufenden Flächen (15,17; 22,23) des rohrförmigen Teiles (12) und der Kappe (16;25) mindestes eine schraubenförmige Nut aufweist, die eine Verbindung zwischen dem Innern des rohrförmige Teiles und der Außenluft herstellt, und daß mindestens eine der beiden Flächen (15,17,; 22,23) ein das Blut koagulierendes Material aufweist.

2. Entlüftungsventil nach Anspruch 1, dadurch gekennzeichnet, daßd die Außenfläche (15) des rohrförmigen Teiles glatt ist und daß die Nut an der mit dieser Außenfläche (15) zusammennwirkenden Innenfläche (17) der Kappe (16) vorgesehen ist.

3. Entlüftungsventil nach Anspruch 1, dadurch gekenn zeichnet, daß das rohrförmige Teil (12) eine glatte Innenfläche (22) aufweist und daß die Nut an der Außenwand eines von der Stirnwad (18) der Kappe (25) abstehenden Zapfens (24) vorgesehen ist.

4. Entlüftungsventil nach einem der Ansprüche 1 bis 3, dadurch gekennzeichnet, daß die Nut eine Tiefe von etwa 1/10 mm aufweist.

5. Entlüftungsventil nach einem der Ansprüche 1 bis 4, dadurch gekennzeichnet, daß die zusammenwirkenden Flächen (15,17; 22,23) zylindrisch bzw. konisch sind.

6. Entlüftungsventil nach einem der vorhergehenden Ansprüche, dadurch gekennzeichnet, daß die Kappe (25) einen das rohrförmige Teil (24) mit radialem Abstand umgebenden Mantel (26) aufweist.

## Claims

1. Air vent as a blood arrester for medical conduits or cannulae,comprising at least one capillary air duct, characterized in that a tubular element (12) whose inside (14) communicates with the conduit or cannula is closed by a caP (16;25), that one of two coacting circumjacent faces (15,17; 22,23) of the tubular element (12) and of the cap (16;25) extends coaxially with the tubular element or cap and contains at least one helical groove which establishes a connection between the inside of the tubular element and the outside air and that at least one of the two faces (15,17; 22,23) contains a blood-coagulating substance.

2. Air vent according to claim 1, characterized in that the outer surface (15) of the tubular element is smooth and that the groove is provided at the inner face (17) of the cap (16) cooperating with said outer surface (15).

3. Air vent according to claim 1, characterized in that the tubular element (12) has a smooth inner surface (22) and that the groove is provided at the outer wall of a pin (24) projecting from the end wall (16) of the cap (25).

4. Air vent according to one of claims 1 to 3, characterized in that the depth of the groove is about 1/10 mm.

5. Air vent according to one of claims 1 to 4, characterized in that the cooperating faces (15,17; 22,23) are cylindrical or conical.

6. Air vent according to one of the preceding claims, characterized in that the cap (25) comprises a jacket (26) encompassing the tubular element (24) in spaced radial relationship.

## Revendication

1. Valve d'évacuation d'air avec barrière sanguine, pour des tuyauteries ou canules à usage médical, comportant au moins un canal capillaire d'évacuation d'air; caractérisée en ce qu'elle comporte une partie tubulaire (12), dont l'intérieur (14) communigue avec la tuyauterie ou la canule, et gui est fermée par un chapeau (16; 25); en ce gue l'une des deux surfaces de révolution (15, 17; 22, 23) de la partie tubulaire (12) et du chapeau (16; 25), qui coopèrent l'une avec l'autre et s'étendent respectivement de manière coaxiale par rapport à la partie tubulaire et par rapport au chapeau, porte au moins une gorge hélicoidale gui met en communication l'intérieur de la partie tubulaire avec l'air extérieur; et en ce gu'au moirs l'une des deux surfaces précitées (15) 17; 22,23) comporte une matière qui provogue la coagulation du sang.

2. Valve selon la revendication 1,caractérisée en ce gue la surface extérieure (15) de la partie tubulaire est lisse; et en ce gue la gorge est prévue sur la surface interne (17) du chapeau (16) gui coopère avec la surface extérieure précitée (15).

3. Valve selon la revendication 1, caractérisée en ce gue la partie tubulaire (12) présente une surface interne (22) gui est lisse, et en ce gue la gorge est prévue sur la surface extérieure d'un bossage (24) en saillie sur le fond (18) du chapeau (25).

4. Valve selon l'une des revendications 1 à 3 caractérisée en ce gue la gorge a une profondeur d'environ 1/10 mm.

5. Valve selon l'une des revendications 1 à 4, caractéerisée en ce gue les surfaces (15,17; 22, 23) gui coopèrent l'une avec l'autre sont cylindrigues ou conigues.

6. Valve selon l'une des revendications précédentes,caractérisée en ce gue le chapeau (25) comporte une jupe (26),gui entoure la piece tubulaire (24) avec un écartement radial.

FIG.1

FIG.2

FIG.3